# EUROPÄISCHE PATENTSCHRIFT

(11) **EP 0 871 691 B1**
(45) Veröffentlichungstag und Bekanntmachung des Hinweises auf die Patenterteilung: **23.04.2003**
(21) Anmeldenummer: 96933342.6
(22) Anmeldetag: 20.09.1996
(51) Int. Cl.: C11B 3/10, C11C 3/00, A61K 47/44

(54) **VERFAHREN ZUR REINIGUNG VON ALKOXYLIERTEN FETTEN**
PROCESS FOR PURIFYING ALKOXYLATED FATS
PROCEDE DE PURIFICATION DE GRAISSES ALCOXYLEES

(30) Priorität: 28.09.1995 DE 19536165
(43) Veröffentlichungstag der Anmeldung: 21.10.1998
(73) Patentinhaber: BASF AKTIENGESELLSCHAFT, 67056 Ludwigshafen (DE)
(72) Erfinder: DRALLE-VOSS, Gabriele, D-64665 Alsbach-Hähnlein (DE); STÖSSER, Michael, D-67141 Neuhofen (DE); LANG, Siegfried, D-67071 Ludwigshafen (DE); SAUPE, Thomas, D-69207 Sanhausen (DE); ZIPPLIES, Matthias, D-67434 Neustadt (DE)
(86) Internationale Anmeldenummer: EP9604116
(87) Internationale Veröffentlichungsnummer: WO97012017

(56) Entgegenhaltungen:
- EP-A- 0 256 631
- EP-A- 0 645 145
- DATABASE WPI Week 8311 Derwent Publications Ltd., London, GB; AN 83-26281K XP002023805 & JP,A,58 020 152 (HAKUGEN KK) , 5.Februar 1983 & PATENT ABSTRACTS OF JAPAN vol. 7, no. 91 (C-162), 15.April 1983 & JP,A,58 020152 (HAGUKEN:KK), 5.Februar 1983,
- DATABASE WPI Week 8921 Derwent Publications Ltd., London, GB; AN 89-156508 XP002023806 & JP,A,01 099 518 (NIPPON LIGHT METAL KK) , 18.April 1989

## Beschreibung

Die Erfindung betrifft ein Verfahren zur Reinigung von alkoxylierten Fetten durch Behandlung mit einem festen Stoff, alkoxylierte Fette, erhältlich nach diesem Verfahren sowie deren Verwendung.

Alkoxylierte Fette werden für eine Vielzahl von Verwendungszwecken eingesetzt. Ein Verwendungszweck, der zunehmend an Bedeutung gewinnt, ist die Verwendung als Lösungsmittelbestandteil zur Herstellung von Lösungen wasserunlöslicher Wirkstoffe, insbesondere Arzneimittel. Zur Verabreichung von Arzneimitteln ist es in bestimmten Fällen erforderlich, daß die Arzneimittel in stabilen Lösungen vorliegen, z.B. dann, wenn die Arzneimittel infundiert werden sollen.

In der EP 256 631, dem WPI/DERWENT AN 83-26281 K &
JP-A-58 020 152 und dem WPI/DERWENT AN 89-156 508 &
JP-A-1 099 518 ist die Reinigung von "used edible oils" beschrieben, die sich beim Gebrauch, z.B. als Frittiermedium, z.T. zersetzen und recycled werden müssen.

Aus der EP 645 145 A sind Arzneimittelzubereitungen bekannt, die aus einer Lösung eines antineoplastischen Wirkstoffs und einem Lösungsmittel bestehen, wobei das Lösungsmittel alkoxylierte Fette als Co-Solvens oder Lösungsvermittler enthält. Die Arzneimittelzubereitung ist durch Zugabe einer Säure oder eines Salzes oder durch Behandlung des alkoxylierten Fettes mit Aluminiumoxid stabilisiert und zwar dadurch, daß der Carboxylat-Gehalt auf einen bestimmten Wert reduziert wird.

Aus der WO 94/12030 ist es bekannt, daß Arzneimittelzubereitungen, wie sie auch in der EP 645 145 beschrieben sind, durch Zugabe bestimmter Säuren stabilisiert werden können, wobei der resultierende pH-Wert unter 8.1 liegen muß.

Die aus diesem Stand der Technik bekannten Bestandteile von Arzneimittelzubereitungen erfüllen jedoch häufig noch nicht die Anforderungen, die an solche Bestandteile bezüglich der Stabilität der resultierenden Zubereitung gestellt werden.

Es ist daher die Aufgabe der vorliegenden Erfindung, ein Verfahren zur Verfügung zu stellen, mit dem alkoxylierte Fette so gereinigt werden können, daß sie zur Verwendung in Arzneimittelzubereitungen besser geeignet sind.

Gegenstand der Erfindung ist demnach ein Verfahren zur Reinigung von alkoxylierten Fetten durch Behandlung mit einem festen Stoff, das dadurch gekennzeichnet ist, daß als fester Stoff eine Mischung aus Aluminiumoxid und einem Silikat eingesetzt wird.

Mit dem erfindungsgemäßen Verfahren kann nicht nur der Anteil an Verunreinigungen reduziert werden, es ist darüberhinaus auch möglich, den Wassergehalt zu verringern und den pH-Wert abzusenken.

Überraschenderweise wird durch das erfindungsgemäße Verfahren auch die Viskosität des alkoxylierten Fettes erniedrigt, was zu besser zu applizierenden Produkten führt. Liegen die Viskositäten des Ausgangsproduktes im Bereich von 700-850 mPa·s, so weist das erfindungsgemäß gereinigte Produkt eine Viskosität im Bereich von 600-750 mPa·s auf, d.h. jeweils eine Reduzierung von 100 mPa·s. Außerdem läßt sich eine Reduzierung von Katalysatorrückständen erreichen.

Eine bevorzugte Ausführungsform des erfindungsgemäßen Verfahrens besteht darin, daß die alkoxylierten Fette mit dem Aluminiumoxid und dem Silikat (Adsorbentien) vermischt und gerührt und anschließend die festen Stoffe abfiltriert werden. Es war überraschend, daß der angestrebte Reinigungseffekt schon durch einfaches Vermischen der Komponenten, ggf. bei erhöhter Temperatur, erreicht wird. Die Behandlung des alkoxylierten Fettes erfolgt bevorzugt durch Mischen mit den Adsorbentien und Rühren bei Temperaturen von 15-180°C vorzugsweise bei 30-100°C, insbesondere 40-80°C. Während der Reaktion kann ein Unterdruck von 10-800 mbar angelegt werden. Die Reaktion kann auch unter Schutzgas (z.B. N₂) durchgeführt werden. Die Reaktionszeit liegt bevorzugt zwischen 1 und 12 h. Der Zusatz der Adsorbentien liegt bevorzugt bei 0,5-15 Gew.-%, insbesondere 1-10 Gew.-%. Es können zusätzlich noch 0-3 Gew.-% eines Filtrierhilfsmittel, wie z.B. Diatomeenerde (Kieselgur) zugegeben werden.

Als Silikate werden bevorzugt Alkali- und/oder Erdalkalisilikate, insbesondere Mg-Silikat oder Ca-Silikat, oder Al-Silikate, aber auch Bleicherden eingesetzt. Als Handelsprodukte seien beispielsweise Ambosol, Tonsil FF oder Magnesol genannt.

Die Mischung von Aluminiumoxid und Silikat wird in der Regel durch mechanische Vermischung der beiden Bestandteile erhalten, z.B. im Reaktionsgefäß.

Eine bevorzugte Ausführungsform des Verfahrens besteht weiterhin darin, daß vor, während oder nach der Behandlung mit den festen Stoffen eine Säure zugesetzt wird. Das gereinigte Endprodukt kann z.B. mit kurzkettigen (1-3 C-Atome) organischen Säuren z.B. Essigsäure auf einen pH-Wert <7 angesäuert werden. Aber auch andere Säuren, wie Citronensäure oder Mineralsäuren, sind geeignet. Die möglichen Säuren sind in der EP 645 145 angegeben. Darüberhinaus können zum Ansäuern auch saure Salze wie das Ammoniumsalz von Aminosäuren, z.B. Glycin-Hydrochlorid eingesetzt werden.

Die vorliegende Erfindung betrifft auch gereinigte alkoxylierte Fette, die nach dem erfindungsgemäßen Verfahren hergestellt wurden, mit einem Gehalt an freien Fettsäuren von 0,03 % oder weniger, sowie die Verwendung dieser alkoxylierten Fette zur Herstellung von Arzneimittelzubereitungen.

Alkoxylierte Fette, die nach dem erfindungsgemäßen Verfahren gereinigt werden, sind zum Beispiel ethoxylierte Fettsäuren, insbesondere ethoxyliertes Ricinusöl, wie es unter dem Handelsnamen Cremophor® EL von der Anmelderin vertrieben wird. Aber auch andere Fette, wie sie in der EP 645 145 A genannt sind, können mit dem erfindungsgemäßen Verfahren gereinigt werden.

Arzneimittelzubereitungen, die mit dem erfindungsgemäß gereinigten und u.U. angesäuerten alkoxylierten Fetten zubereitet werden, sind insbesondere solche mit wasserunlöslichen Wirkstoffen. Die Zubereitungen enthalten in der Regel noch einen weiteren Lösungsmittelbestandteil, wie eine Alkohol, insbesondere Ethanol, wobei das Verhältnis von alkoxyliertem Fett und Alkohol 30:70 - 70:30 betragen kann.

Als Wirkstoffe werden vor allem wasserunlösliche Wirkstoffe vorgesehen, insbesondere antineoplastische Wirkstoffe wie z.B. Paclitaxel (Taxol®). Bezüglich der anderen möglichen Wirkstoffe (wie Teniposide, Camptothecin und deren Derivate) wird ausdrücklich auf die EP 645 145 A Bezug genommen.

### Beispiele

### Beispiel 1

Es wurde ein ethoxyliertes Rizinusöl (Cremophor® EL) mit folgender Analytik eingesetzt:
- pH-Wert 7,0
- Viskosität : 750 mPa·s
- Wassergehalt nach Karl-Fischer ca. 2.5 %
- Anteil freier Fettsäuren 0.1-0.5 %

2500 kg 100 %iges Cremophor® EL wurde unter N₂ auf 50°C erhitzt und mit 75 kg Magnesiumsilikat (Ambosol 500 der Fa. Hoechst), 75 kg saurem Aluminiumoxid sowie 5,25 kg Diatomeenerde (Hyflow der Fa. Lehmann u. Voss,=Kieselgur-Filtrierhilfsmittel (flußkalzinierte weiße Süßwasserdiatomeen)) versetzt. Die Mischung wurde 3,5 h bei 50 bis 55° gerührt. Anschließend wurden die Adsorbentien abfiltriert. Die Porengröße des Filters lag zwischen 10 µm und 20 µm.

### Beispiel 2

Es wurde wie in Beispiel 1 verfahren, statt des sauren Al-Oxid wurde jedoch ein basisches Al-Oxid eingesetzt.

### Ergebnisse:

| | Adsorbent | H₂O-Gehalt % | Gehalt an freien Fettsäuren % |
|---|---|---|---|
| 1 | 3 % Magnesiumsilikat/ | 0,15 | 0,03 |
| | 3 % saures Aluminiumoxid ¹⁾ | | |
| 2 | 5 % Magensiumsilikat/ ²⁾ | 0,49 | 0,02 |
| | 5 %basisches Aluminiumoxid | | |

| | | | |
|---|---|---|---|
| ¹⁾ Aluminiumoxid 90 aktiv, sauer, Aktivitätsstufe 1 der Fa. Merck | | | |
| ²⁾ Al₂O₃-W 200 stark basisch | | | |

Der pH-Wert des gemäß Beispiel 1 erhaltenen Produkts lag bei 5,9, d.h. bereits ohne zusätzliches Ansäuern wird durch das Absenken des pH-Werts ein stabilisierender Effekt erreicht (Messung des pH-Werts in 10%iger wäßriger Lösung.)

Die Viskosität lag bei 650 mPa·S

Zum Vergleich wurden folgende Versuche durchgeführt:

| | Adsorbent | H₂O-Gehalt % | Gehalt an Fettsäuren % |
|---|---|---|---|
| 3 | 3 % Magensiumsilikat | 0,69 | 0,11 |
| 4 | 5 % Magnesiumsilikat | 0,16 | 0,11 |
| 5 | 3 % saur. Al-oxid ¹ | 0,24 | 0,06 |

Die Ergebnisse zeigen, daß bei Verwendung von Aluminiumoxid oder Silikat jeweils einzeln die erwünschten Ergebnisse, insbesondere der niedrige Gehalt an Fettsäuren, nicht erreicht werden. Die erfindungsgemäße gemeinsame Verwendung der Adsorbentien führt jedoch in überraschender Weise zu einem synergistischen Effekt, so daß ein Produkt erhalten wird, das in vorteilhafter Weise für Arzneimittelzubereitungen geeignet ist.

## Patentansprüche

1. Verfahren zur Reinigung von alkoxylierten Fetten durch Behandlung mit einem festen Stoff, **dadurch gekennzeichnet, daß** als fester Stoff eine Mischung aus Aluminiumoxid und einem Silikat eingesetzt wird.

2. Verfahren nach Anspruch 1, **dadurch gekennzeichnet, daß** die Behandlung bei 15-180°C vorgenommen wird.

3. Verfahren nach Anspruch 1, **dadurch gekennzeichnet, daß** als Silikat ein Alkali- oder/und Erdalkalisilikat verwendet wird.

4. Verfahren nach Anspruch 1, **dadurch gekennzeichnet, daß** die Behandlung darin besteht, daß die alkoxylierten Fette mit dem Aluminiumoxid und dem Silikat vermischt und gerührt und anschließend die festen Stoffe abfiltriert werden.

5. Verfahren nach Anspruch 4, **dadurch gekennzeichnet, daß** 0,5 bis 15 Gew.-% der festen Stoffe, bezogen auf die Gesamtmischung, eingesetzt werden.

6. Verfahren nach Anspruch 4, **dadurch gekennzeichnet, daß** bei der Behandlung zusätzlich 0-2 Gew.-% eines Filtrierhilfsmittels zugegeben wird.

7. Verfahren nach Anspruch 1, **dadurch gekennzeichnet, daß** vor, während oder nach der Behandlung eine Säure zugesetzt wird.

8. Gereinigte alkoxylierte Fette, erhältlich nach dem Verfahren gemäß Ansprüchen 1 bis 7, mit einem Gehalt an freien Fettsäuren von 0,03 % oder weniger.

9. Verwendung von gereinigten alkoxylierten Fetten gemäß Anspruch 8 zur Herstellung von Arzneimittelzubereitungen.

## Claims

1. A process for purifying alkoxylated fats by treatment with a solid substance, wherein a mixture of aluminum oxide and a silicate is employed as solid substance.

2. A process as claimed in claim 1, wherein the treatment is carried out at 15-180°C.

3. A process as claimed in claim 1, wherein an alkali metal and/or alkaline earth metal silicate is used as silicate.

4. A process as claimed in claim 1, wherein the treatment comprises mixing and stirring the alkoxylated fats with the aluminum oxide and the silicate, and subsequently filtering off the solid substances.

5. A process as claimed in claim 4, wherein from 0.5 to 15 % by weight of the solid substances, based on the complete mixture, are employed.

6. A process as claimed in claim 4, wherein additionally 0-2 % by weight of a filtration aid is added in the treatment.

7. A process as claimed in claim 1, wherein an acid is added before, during or after the treatment.

8. A purified alkoxylated fat obtainable by the process as claimed in any of claims 1 to 7 with a free fatty acid content of 0.03% or less.

9. The use of a purified alkoxylated fat as claimed in claim 8 for the production of pharmaceutical formulations.

## Revendications

1. Procédé pour la purification de graisses alcoxylées par traitement avec une substance solide, **caractérisé par le fait qu'**on utilise comme substance solide un mélange d'oxyde d'aluminium et d'un silicate.

2. Procédé selon la revendication 1, **caractérisé par le fait que** le traitement est effectué à 15-180°C.

3. Procédé selon la revendication 1, **caractérisé par le fait qu'**on utilise comme silicate un silicate alcalin et/ou alcalino-terreux.

4. Procédé selon la revendication 1, **caractérisé par le fait que** le traitement consiste à mélanger et à agiter les graisses alcoxylées avec l'oxyde d'aluminium et le silicate, et ensuite à séparer les substances solides par filtration.

5. Procédé selon la revendication 4, **caractérisé par le fait qu'**on utilise 0,5 à 15% en poids de substances solides, par rapport au mélange total.

6. Procédé selon la revendication 4, **caractérisé par le fait qu'**on ajoute en outre, lors du traitement, 0-2% en poids d'un adjuvant de filtration.

7. Procédé selon la revendication 1, **caractérisé par le fait que** l'on ajoute un acide avant, pendant ou après le traitement.

8. Graisses alcoxylées purifiées, pouvant être obtenues conformément au procédé selon les revendications 1 à 7, avec une teneur en acides gras libres de 0,03% ou moins.

9. Utilisation des graisses alcoxylées purifiées selon la revendication 8 pour la préparation de compositions médicamenteuses.
